# EUROPEAN PATENT APPLICATION

(11) **EP 4 111 877 A1**
(43) Date of publication of application: **04.01.2023**
(21) Application number: 22181970.9
(22) Date of filing: 29.06.2022
(51) Int. Cl.: A24F 40/00, A61M 15/06

(54) **METHOD FOR TRACKING SMOKING ACTIVITY OF TOBACCO CIGARETTE USERS, CORRESPONDING SYSTEM AND COMPUTER PROGRAM PRODUCT**

(30) Priority: 02.07.2021 IT 202100017567
(71) Applicant: Eclat S.r.l., 95123 Catania CT (IT)
(72) Inventor: POLOSA, Riccardo, 95123 Catania (IT)
(74) Representative: Crovini, Giorgio

(57) **Abstract**

A method for tracking activity of tobacco cigarette users (11) making use of tobacco cigarettes and/or nicotine delivery systems, said use being tracked by a controlling entity (15), comprising, upon submission of a tobacco cigarette user identifier (ID) at a user smoker terminal (12), performing a self-reporting (300) of tobacco cigarette user activity information (Q) comprising information (NCC, NDC) regarding the smoking activity in a given period, in particular daily, in particular in the day before the self-reporting, upon execution of said self-reporting (300) sending (420) corresponding tobacco cigarette user activity information (Q) comprising information (NCC, NDC) regarding the smoking activity in a given period from the user smoker terminal (12) to a remote computer server (14) through a communication link (13), storing said tobacco cigarette user activity information (Q) comprising information (NCC, NDC) regarding the smoking activity in a given period from the user smoker terminal (12) in a database (14b) at the remote server (14), performing (520) one or more control procedures (600, 700, 800) on the basis of the tobacco cigarette user activity information (Q) comprising information (NCC, NDC) regarding the smoking activity in a given period from the user smoker terminal (12) stored in said database (14), at least one of said one or more control procedures (600) including evaluating a number (NCC) of cigarettes smoked in the given period, in particular daily, evaluating said number of cigarettes (NCC) and in case said number of cigarettes (NCC) does not match a given criterion, generating (520) at the server (14) a corresponding alert (620), sending (530) said alert (620) to a controlling entity terminal (15).

## Description

### Technical field

The present description relates to techniques for tracking smoking activity of tobacco cigarette users.

Various embodiments may apply e.g., users of conventional tobacco cigarettes (in the following also indicated as TCs) and/or new generation nicotine delivery devices (in the following also indicated as NDDs).

### Description of the prior art

Smoking cessation and smoking switching protocols entail TCs users to adhere to specific health/research programs, which require recording of the amount of use of TCs and/or NDDs, with particular emphasis on smoking reduction, smoking cessation or smoking substitution via switching to NDDs use.

Compliance with smoking cessation and/or smoking substitution (i.e. switching research protocols) is important because non-compliance in different degrees may decrease or nullify the anticipated improvements in study endpoints and in general health. However, tracking compliance is a difficult task for the health care practitioner (HCP), engaging with TCs users enrolled in smoking cessation and/or smoking substitution (i.e. switching trials). A close tracking by the HCP is impracticable because careful verification of smoking activity would entail attending a large number of visits at the clinical site

### Object and summary

An object of one or more embodiments is to provide a method for tracking smoking activity of tobacco cigarette users that solves the drawbacks of the prior art allowing a close tracking of tobacco cigarettes users.

According to one or more embodiments, that object is achieved thanks to a method having the characteristics specified in Claim 1. One or more embodiments may refer to a corresponding computer system performing the method and to a computer program product that can be loaded into the memory of at least one computer and comprises parts of software code that are able to execute the steps of the method when the product is run on at least one computer. As used herein, reference to such a computer program product is understood as being equivalent to reference to a computer-readable means containing instructions for controlling the processing system in order to co-ordinate implementation of the method according to the embodiments. Reference to "at least one computer" is evidently intended to highlight the possibility of the present embodiments being implemented in modular and/or distributed form.

The claims form an integral part of the technical teaching provided herein in relation to the various embodiments.

According to the solution described herein, it is described a method for tracking activity of tobacco cigarette users making use of tobacco cigarettes and/or nicotine delivery systems, said use being tracked by a controlling entity, comprising,
upon submission of a tobacco cigarette user identifier at a user smoker terminal, performing a self-reporting of tobacco cigarette user activity information comprising information regarding the smoking activity in a given period, in particular daily, in particular in the day before the self-reporting,
upon execution of said self-reporting sending corresponding tobacco cigarette user activity information comprising information regarding the smoking activity in a given period from the user smoker terminal to a remote computer server through a communication link,
storing said tobacco cigarette user activity information comprising information regarding the smoking activity in a given period from the user smoker terminal in a data base at the remote server,
performing one or more control procedures on the basis of the tobacco cigarette user activity information comprising information regarding the smoking activity in a given period from the user smoker terminal stored in said data base, at least one of said one or more control procedures including evaluating a number of cigarettes smoked in the given period, in particular daily,
evaluating said number of cigarettes and in case said number of cigarettes does not match a given criterion, generating at the server a corresponding alert,
sending said alert to a controlling entity terminal.

In variant embodiments, said server includes a web interface accessible from said controlling entity terminal, said web interface being configured to display data stored of said database or processing of said data and/or display said alert, upon access.

In variant embodiments, said sending said alert to a controlling entity terminal is performed by push notification.

In variant embodiments, said information regarding the smoking activity in a given period includes information regarding usage of an electronic nicotine delivery system.

In variant embodiments, said evaluating if said number of cigarettes matches a given criterion includes evaluating a variation of said number of cigarettes with respect to a reference baseline number of smoked cigarettes, in particular stored during an initialization phase before the first self-reporting operation,

In variant embodiments, said evaluating if said number of cigarettes matches a given criterion includes evaluating if a reduction of the smoked number of cigarettes in the given period with respect to the baseline number greater of a given threshold value occurs.

In variant embodiments, the method includes issuing an alert message if said reduction occurs in an observation period comprising a plurality of given periods, in particular a number of consecutive given periods and/or a number of periods between or from a determined event, in particular a medical visit.

In variant embodiments, said self-reporting includes reporting whether the tobacco cigarette user has performed a glycemia check in the given period.

In variant embodiments, said self-reporting includes self- reporting whether the tobacco cigarette user has reported a health status in the given period.

The solution here described refers also to a system for tracking activity of tobacco cigarette users making use of tobacco cigarettes and/or nicotine delivery systems, said use being tracked by a controlling entity comprising
at least a user terminal comprising a front-end of a software application configured for inputting user data,
a remote server comprising a back-end of a software application coupled through a communication link to said front end application in the user terminal and a data base accessible for writing or reading data by the back-end application,
said system being configured to perform the method of any of the previous embodiments.

In variant embodiments, said back-end application comprises a user interface to access, from the node and/or a terminal external to said node , inputted user data and/or processed user data and to issue signals, obtained from said inputted user data and/or processed user, which are also accessible on said user interface,

The solution here described refers also to a computer program product that can be loaded into the memory of at least one computer and comprises parts of software code that are able to execute the steps of the method of any of of any of the previous embodiments when the product is run on at least one computer.

### Brief description of the drawings

The invention will now be described purely by way of a non-limiting example with reference to the annexed drawings, in which:
- Figure 1 represent schematically an architecture of a system implementing the method here described;
- Figure 2 represents a flow diagram of a possible embodiment of the method here described;

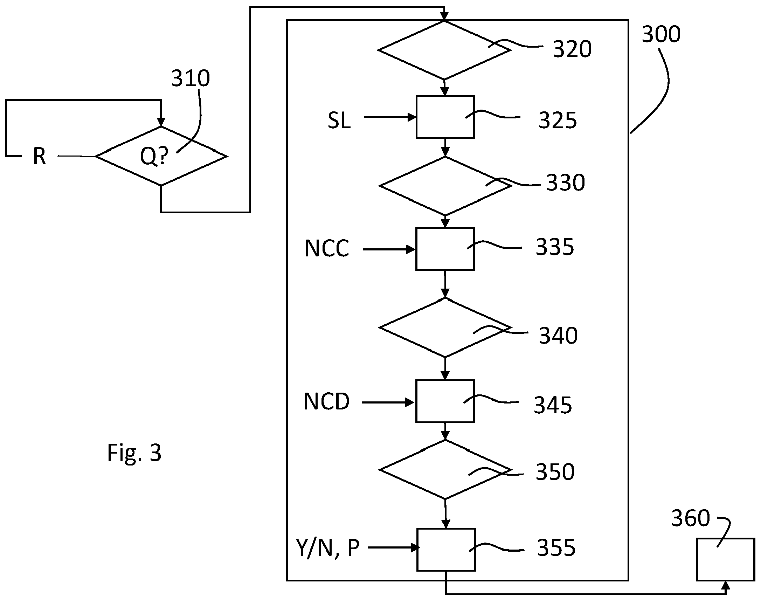

- Figure 3 represents a flow diagram detailing an operation of the method of Figure 2;
- Figures 4-10 represent schematically computer display visualization of input forms used by the operations of the method of Figure 2;
- Figures 11 and 12 represent schematically notification displayed at a user terminal according to the method of Figure 2;
- Figure 13 represents a first example of control procedure according to the method of Figure 2;
- Figure 14 represents a second example of control procedure according to the method of Figure 2;
- Figure 15 represents a third example of control procedure according to the method of Figure 2.

### Detailed description of embodiments

The ensuing description illustrates various specific details aimed at an in-depth understanding of the embodiments. The embodiments may be implemented without one or more of the specific details, or with other methods, components, materials, etc. In other cases, known structures, materials, or operations are not illustrated or described in detail so that various aspects of the embodiments will not be obscured.

Reference to "an embodiment" or "one embodiment" in the framework of the present description is meant to indicate that a particular configuration, structure, or characteristic described in relation to the embodiment is comprised in at least one embodiment. Likewise, phrases such as "in an embodiment" or "in one embodiment", that may be present in various points of the present description, do not necessarily refer to the one and the same embodiment. Furthermore, particular conformations, structures, or characteristics can be combined appropriately in one or more embodiments.

The references used herein are intended merely for convenience and hence do not define the sphere of protection or the scope of the embodiments.

The solution here described refers to a method and system for close tracking of tobacco cigarettes, TCs, consumption and/or nicotine delivery devices, NDDs, (e.g. e-cigarettes or heated tobacco products) by tobacco cigarette users, e.g. participant to cessation or switching protocols; such participants, according to the method here described, may report, for instance on a daily basis, the consumption of cigarettes and use of nicotine delivery devices via a user terminal comprising a front end software application communicating with a corresponding back-end application TA at a remote server, which stores smoking tracking data per each user at said remote server and on the basis of such smoking data performs violation controls, issuing alerts if a violation occurs.

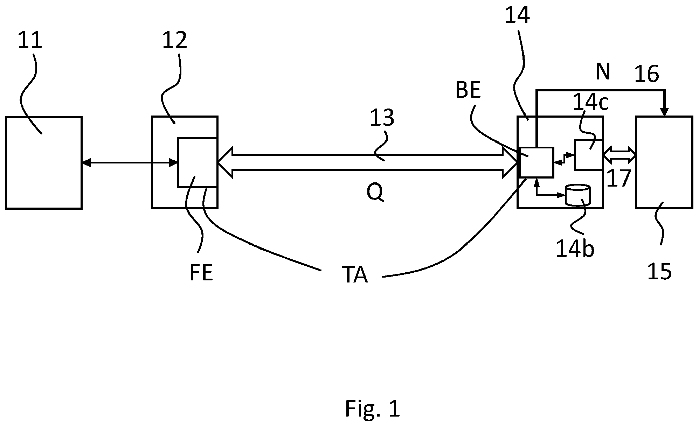

Therefore, in figure 1 is described the architecture of a system for implementing the method for tracking smoking activity here described.

The system as whole is indicated with the numerical reference 10. With the number 11 is indicated a tobacco cigarette user, e.g. a participant to smoking cessation and smoking switching protocols or also simply a smoker which smoking activity needs tracking by a HCP, e.g. a doctor or a physician. The tobacco cigarette user 11 is equipped with a user equipment 12, which may for instance a mobile terminal, configured to run a front-end FE of a software application TA, e.g. a mobile application or app, and to communicate, through a communication link 13, with a remote node 14 which can communicate with other terminals 15, i.e., controller terminals, equipped with the front-end FE of the application TA, i.e. a terminal which comprises computer functions, including input interfaces, such as keyboards and/or microphone, and output interfaces, such as speakers and displays, as well as computer and means for running software applications, such as a operative system, e.g. Android or iOS, memory registers and non-volatile memory means, and further comprises wireless transceiving means to establish such a communication link 13, e.g. a 4G or 5G communication link, although clearly other type of links may established, through Wi-Fi and other communication networks. For instance, the user equipment 12 may be a smartphone, with an Android or iOS operating system.

The remote node 14 or server, includes a back-end software application BE - i.e., a server software application representing the back-end of the application TA, which front-end FE is stored in the user equipment 12 - which exchanges data over the communication link 13 with the front-end FE of the application TA in the user equipment 12.

The communication link 13 is preferably a mobile communication network, e.g. a 5G communication network, although it can be represented by any communication network or set of interoperating communication with which the user terminal 11 is configured to exchange data, using its communication interfaces, e.g. one of Wi-Fi, Bluetooth, IrDA interfaces coupling to hotspot or routing devices accessing networks which are linked to the server 14.

A database 14b, i.e., a module for storing data in a structured manner, in particular, as a structure of records comprising fields, may also be present in the node 14, to store data received by the back-end application BE, and a web interface 14c which can be accessed by the controller terminals 15, in particular through a respective controller link 17, which can be represented by the same network which implements the link 13 or it can be implemented differently. The controller terminals 15 are used by the HCPs to access a portion of the back-end application BE, represented as a dashboard trough the web interface 14c, which allows the HCP to review the user activity data and to receive alerts from the application TA regarding such activity. Such a dashboard is a graphic interface configured as dashboard with indicators regarding the user behaviour based on the data of reports in the database 14b processed by the bac-end BE and on the alerts and signaling issued by the back-end BE, for instance control showing alerts and also graphs showing the user behavior, e.g. number NCC, over time. Also, the information in the database 14b can be processed considering a population of users, e.g. computing statistical values and corresponding graph representations.

As shown in figure 1, a further notification link 16 is provided which allows the backend application BE in the server 14 to send notification message N directly to the controller terminal 15 of the HCP. Such notification link 16 can be a mobile communication network, on which notification messages are sent, or can be any other communication network allowing the server node 14 and controller terminal 15 to communicate, in particular point to point. The notification link 16, the communication link 13 and the controller link 17 may use a same network or part of a same network. The notification message N in embodiment are push notifications, although the method here described may use different system of messaging.

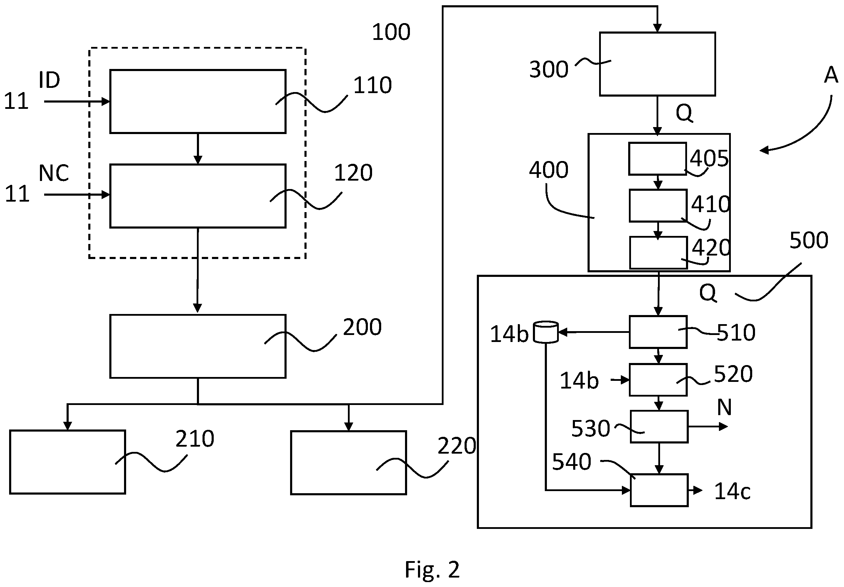

In figure 2 it is shown a flow diagram schematizing the operations of an embodiment of the application TA. Such embodiment is directed in particular to diabetic tobacco cigarette users, hence the check on the glycemia as indicated in the following.

Thus, the application TA includes a first login procedure 100 which in turn includes a first step 110 of entering a unique subject ID (identification) - which is provided for instance by the HCP, e.g. a study Principal Investigator.

Then in a step 120 is entered a baseline number NC of cigarettes smoked in the determined period, e.g. daily. Such baseline number NC is a reference number of cigarettes which the tobacco cigarette user 11 has indicated as its usual average number of cigarettes, in the determined period, e.g. daily, at a baseline time, e.g. at the time of entering a cessation or smoking protocol, and with respect to which for instance a reduction in smoking is calculated.

Completion of the login procedure with identification ID and number NC gives access to a step counting authorization screen, which can authorize in a step 200 for an activity tracking which can include a function of steps counting 210. Optionally, walking plus Running Distance" functions 220 calculating distance on the basis of step count may be included .

In particular after a given time, e.g. a day, after installation of the front-end FE of the application TA on their user equipment 11, e.g. smartphone, the tobacco cigarette user 11 after login and optional step 200 is then entered in a self-reporting procedure 300, which outputs a self-report Q, e.g. a questionnaire comprising one or more question which answer represent a quantitative indication of the daily smoking activity. It is here underlined that such daily smoking activity refers preferably to the activity during the day before, rather that during the current day, i.e. to the activity particular in the day before the day the self-reporting is taking place. This also explains why the application TA is preferably operative after a day from installation.

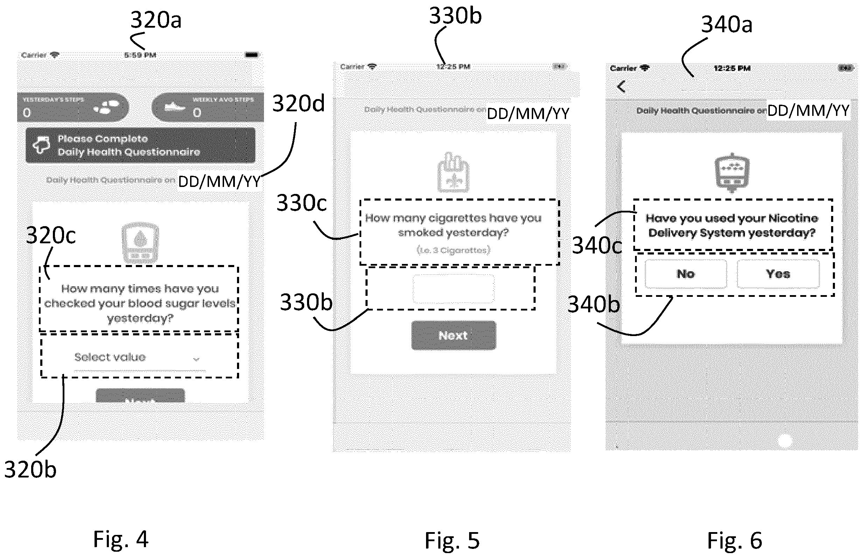

Such a self-reporting procedure 300 may comprise an initial reminding step 310 in which the self-reporting procedure checks a status of completion of the daily self-report Q and if the daily self-report Q is incomplete issues a reminder R, e.g., a pop-up reminder message on the display of the terminal 12, to the user 11 asking to perform the ensuing self-report filling procedure 320, 330, 340 of the daily self-report Q. The reminding step 310, if the daily self-report Q is incomplete, is repeated at each access to the procedure 300 or periodically throughout the day within the execution of the application TA until the tobacco cigarette user 11 has completed all the answers required by the daily self-report Q. In the daily self-report Q filling procedure 320, 330, 340 tobacco cigarette users 11 are requested to report if they have checked:
- blood sugar level SL in a step 320. This can be performed using a form 320a, displayed on the graphical interface of the terminal 12, shown in figure 4, which in the example shown asks, displaying a request of input 320c, specifying for instance to introduce how many times the blood sugar level SL has been checked the day before, selecting a value from a drop-down menu 320b. This facilitates data insertion by the user 11, in particular if the terminal 11 does not include a keyboard or the keyboard is difficult to use, although in variant embodiments the user can directly type the value into a field (or use other input means available in the terminal 11) . An indication 320d of the day, month and year to which the form 320 refers may be present in such form and in the form 330a, 340a, 350a described in the following. Alternatively, step 320 may ask simply if the user has checked the blood (glucose) sugar level, yes or no (see also description of procedure 700 in the following). As also discussed in the following, step 320 may be optional;
- a number of cigarettes smoked NCC in a step 330, using a form 330a, shown in figure 5, displayed on the graphical interface of the terminal 11, which in the example shown asks, displaying a request of input 330c, specifying for instance to introduce how many cigarettes has been smoked the day before, in this case using the input method of an introduction of a value in a field 330b, in a free text input filed, (other forms of form input, such as drop-down menu can be used, alternatively);
- a usage NCS of nicotine delivery device or NDD in a step 340, in a form 340a, shown in figure 6, displayed on the graphical interface of the terminal 11, which in the example shown ask, displaying a request of input 340c, specifying for instance to answer if nicotine delivery device NCD has been used, with a yes/no button interface 340b, or employing another input method to select an option, which may also include a following form or a field to specify the number of uses, by the already described input methods;
- a further optional request may be presented in a step 350, asking the user 11 to report about potential health problems. In a corresponding form 350a in figure X+3, in a request 350c the user 11 is asked to specify if a health problem has occurred the day before (or on a specific day), and yes/no option input means 350b are provide there, along with a drop-down menu input 350d, to indicate the health problem, in case of affirmative reply in yes/no option input means 350. Users 11 may have also the option to use the front-end FE of the application TA to perform a call for medical assistance if necessary, for instance by a call button provided in the same form 350a, which may call a pre-selected number corresponding to the medical assistance using the mobile communication capabilities of the terminal 12.

As mentioned, the self-report Q comprises at least a request of information regarding the smoking activity in a given period, in the example daily, but the periodicity of the self-report Q may be different, e.g. each two days. Such information regarding the smoking activity in a given period comprises an information regarding if a smoking activity has taken place in the given period. Additionally, it may include an information including a quantitative indication of the daily smoking activity.

Thus, the self-report may include step 330 and/or 340, with for each step the option additional indication of how many cigarettes or respective use of the NDD are performed. As mentioned, the self-report may optionally include also step 320 and/or step 350.

The order of the steps 320, 330, 340, 350, may be also different with respect to the example of figure 4, and other steps and operations may be included in the procedure 300.

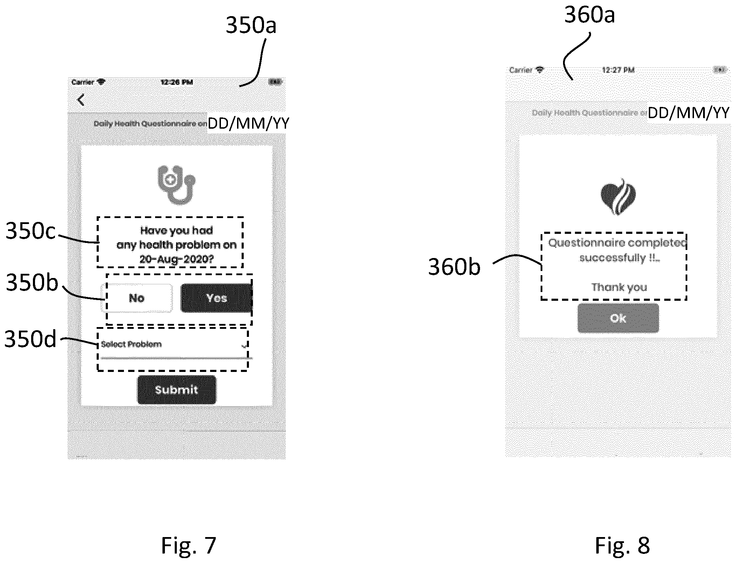

Once completed the self-report Q, users 11 receive an end questionnaire signal 360, for instance they are greeted by a "Thank you" message 360a (as shown in figure 8) on the display of the user equipment 12.

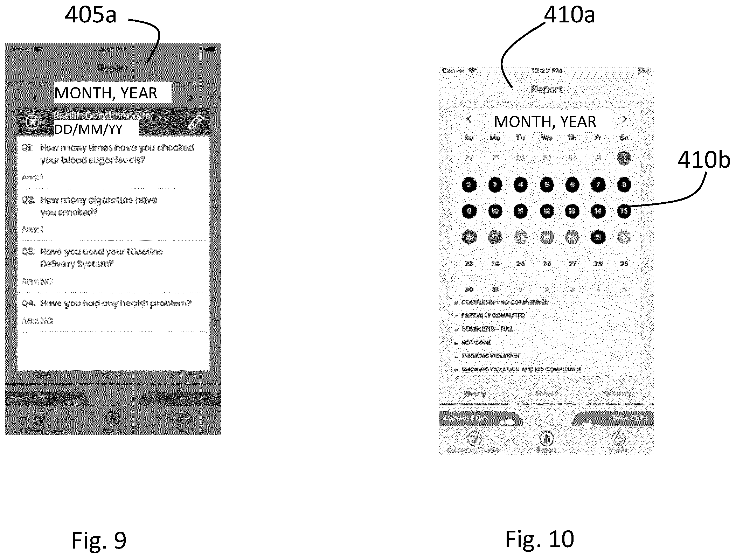

A review procedure 400 follows the self-reporting procedure 300, which includes, as shown in figure 9, a step of answers review 405, in which a form 405a may be shown on the display of the user equipment 12. This may happen also before the end questionnaire signal 360. Form 405a shows the answers to the requests in steps 320-350 and allows the user 11 to review such reply to the requests in steps 320-350. In case of error, the user 11 may access again the self-report Q to correct the answer or answers.

In figure 10 it is shown a calendar form 410a, which may be invoked at step 410 after the filling of the questionnaire 300, which shows a calendar 410b, in month representation configuration, where different status of completion of the self-report Q for the corresponding day are shown by a color code, corresponding for instance to complete with no compliance/partially complete/complete full or also specific violations, e.g. smoking violation or smoking violation and no compliance.

The front-end FE of the application TA after step 400, in particular 405, may send the self-report Q for that, period, e.g. day in a step 420 to the back-end portion BE of the application TA at the server 14 over the communication link 13, as also shown in Figure 1. The back-end portion BE is configured to perform then a back-end procedure 500, which includes storing 510 the daily self-reports Q to in corresponding records of the database 14b which for instance have the identifier ID as indexing field, and comprise fields for the given period to which the self-report Q refers and fields for in the information, e.g. at least field indicating the number of cigarettes smoked NCC and the nicotine delivery usage NCD. Additionally, fields can be devoted to the answers of steps 320 and 260 and of counting 210 and 220. Thus, a record representing a daily self-report Q of a user 11 with an identifier ID, may comprise the fields ID, day, NCC, NCD, filled with the corresponding values in the corresponding received daily self-report Q.

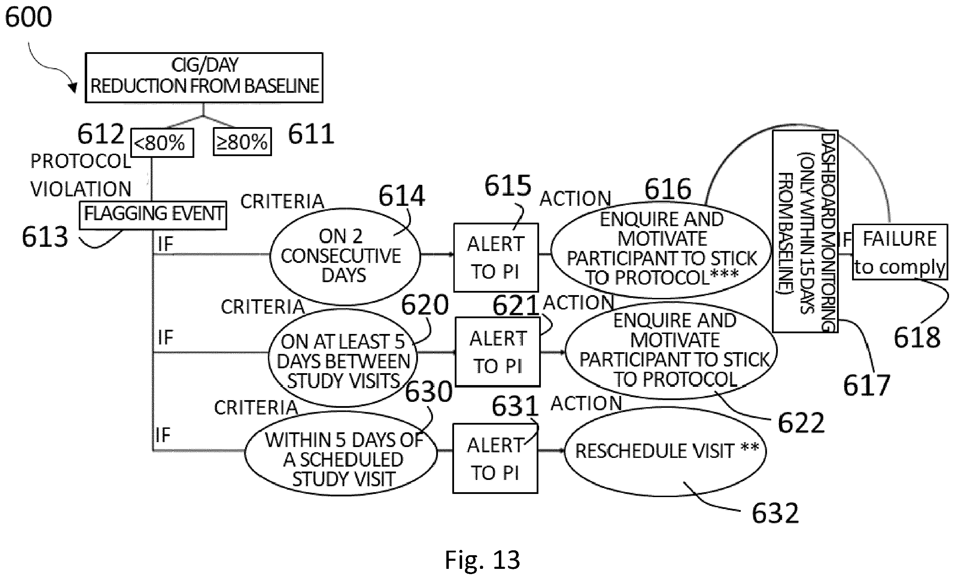

The back-end portion BE of the application TA is also configured to perform, in a step 520 control procedures on the records of the database BE. To this regard, in figure 13 it is shown an example of such control procedures, i.e, a control procedure 600 checking day cigarette reduction with respect to the baseline number NC.

In such control procedure 600, it is evaluated by the back-end portion BE, using the data in database 14b for a given user identifier ID, if the percentage reduction, e.g. the ratio of the number of cigarettes smoked NCC to the baseline number multiplied by 100 is lower than 80% (step 611) or greater than 80% (612), i.e. the number of cigarettes smoked NCC is reduced more than 80% of the baseline number NC. Of course, reduction check of steps 611, 612 can be implemented also with other reduction threshold different from 80% and calculated with other criteria, e.g. simple ratio or other relations to which yield a numerical indication of a variation in number of smoked cigarettes with respect the baseline. In general, it is provided to evaluate the number of cigarettes NCC against a given criterion, in particular, depending from said baseline number NC, in the example a determined valued of percentage reduction of the number of cigarettes NCC with respect to the baseline number NC, and in case (such as in step 611) said number of cigarettes NCC does not match the given criterion a violation may be signaled, as described below. Thus, portion BE also accessed records of previous days for the same ID to perform the reduction comparison.

In case the user 11 answers that is lower that 80%, this represents a protocol violation, thus a flag issue procedure 613 is started which evaluates a plurality of criteria 614, 620, 630. A first criteria check 614 may check if the protocol violation has occurred on two consecutive days (or another number of different consecutive days). In the affirmative an alert 615 is issued to the HCP, which may be prompted with an action 616 to be performed, e.g. enquire and motivate the participant to follow the protocol, then a dashboard monitoring 617 may be activated, for instance only within 15 days from the baseline time, e.g. the beginning of the protocol. Such dashboard monitoring 617 may include monitoring the activity of that specific user through the dashboard, through the web interface 14c, which allows the HCP to review the user activity data and to receive alert from the application TA, in particular from the back-end BE, regarding such activity. The cycle 616-617 is repeated in case it is checked a failure to comply 618.

A second criteria 620 may check if the protocol violation (612) has occurred on at least a given number, e.g. five, of days between study visits. In the affirmative an alert 621 to the controller is issued by notification and an action 622, similar to action 616, e.g. enquire and motivate the participant to follow the protocol, is prompted on the controller terminal 15.

A third criteria 630 may check if the protocol violation (612) has occurred on at least a given number, e.g. five, of days before a scheduled study visit. In the affirmative an alert 631 to the controller is issued and an action 632 of rescheduling the visit may be prompted on the controller terminal 15.

The alerts 615, 621, 631 may be sent in corresponding notifications N in a notification step 530, following step 520, to the controller terminals 15 by the notification link 16.

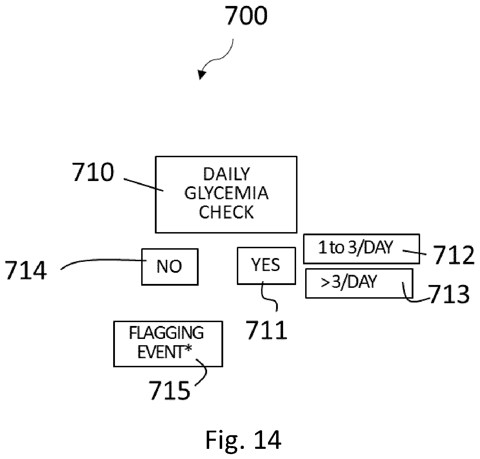

In figure 14 it is shown an optional control procedure, which is a procedure of glycemia check monitoring 700, which requires a daily glycemia check verification 710, i.e. asking the user 11 to input if the blood glucose level has been checked or not over the given period, e.g. daily. This test can be also embodied by the step 320b, which more specifically ask how many times the check has been performed (answer 0, no check, 1 or mor, yes check). Thus, step 710 is performed at the front-end FE, its result, yes/no or number of checks, is send to the back-end application BE for performing the subsequent steps of procedure 700. If this result is positive, 711 it is checked at the back-end if 1 to 3 checks per day have been performed (step 712) or greater than 3 per day (step 713). In the negative, 714, a flag 715 is issued, which determines for instance a notification N on the daily notifications 510a.

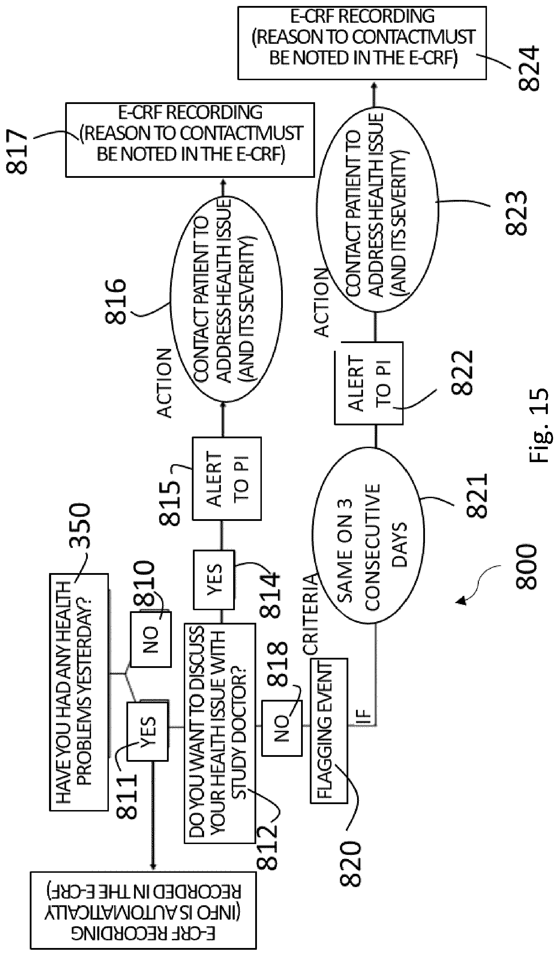

In figure 15 it is shown a control procedure 800 regarding the value in the self-report Q in response to step 350, i.e. indication of health problems. requesting smoking consumption answers, 800.

At step 340 at the front-end FE of the application TA is asked if the user 11 had health problems the day before. If the corresponding value in the daily self-report is negative, step 810, the procedure 800 takes no action. In the affirmative, 811, the information of a problem occurring on a given day is recorded, 812, in a in an electronic medical record of the user 11 and in a step 813 it is asked to the user 11, sending for instance a message through the communication link 13 to the front-end FE of the application TA in the user equipment 12, if a discussion with a HCP is required about the health problem. In the affirmative 814, an alert to the controller 815 is issued, an action 816 is prompted to the controller, in order to contact the user 11 to address health issue and its severity, a recording 817 in an electronic health file of the user 11 is performed with the result of the contact. In the negative, 818, in a step 820 a flag is asserted, which signals anyway the existence of the health problem. Then, in the example a checking of a criterion 821, i.e. if a same flag has been issued on a given number of consecutive days, e.g. 3. If the criterion is complied, an alert 822 is issued an action 823 is prompted, in order to contact the user 11 to address health issue. Of course, other criteria may be possible, or also the flag assertion step 820 may determine directly issued of alert 822.

Also, the alerts of procedures 700 and 800 may be sent in the notification step 530.

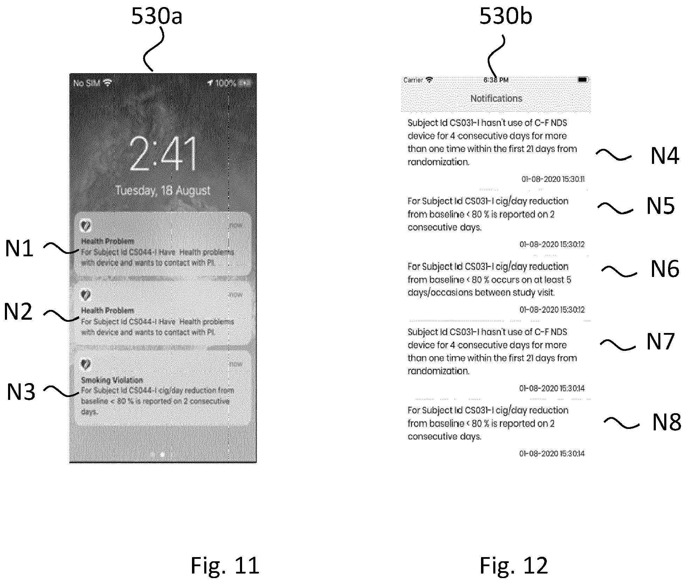

In figures 11 and 12 are shown samples of notifications N at the controller terminal 15, performed in the notification step 530. In figure 11 it is shown a screen 530a of the terminal 12, which shows notifications N1, N2, N3 on the terminal 11. These are push notification, and the back-end application 14b is equipped with a server side service to send push notification to the controller terminal 15 through the notification link 16. For instance, Firebase Cloud Messaging (FCM), a cross-platform cloud solution for messages and notifications for Android, iOS, and web applications, may be used. Notification N1 and N2 for instance indicates that a tobacco cigarette user 11 with an identifier ID signals a health problem (step 350), and notification N3 indicates that the same tobacco cigarette user has committed a violation (alert 614, reduction with respect to baseline insufficient for two days).

In figure 12 other examples of notifications N4, N5, N6, N7, N8 on the controller terminal 15 are shown, in a screen 530b, which represent a list, i.e. a history of all notification, received by a particular controlling entity terminal 15. Notifications N4 e N7 signals at different times that the user 11 has not been using the NDD for a given number of consecutive days more than one time, which is also a criterion which may checked in step 520, on the base of the information gathered in step 330. Notifications N5, N6, N8 signals at different times that the user 11 has occurred in an alert 614, reduction with respect to baseline insufficient for two days.

Thus, in general the method for tracking activity of tobacco cigarette users 11 making use of tobacco cigarettes and/or nicotine delivery systems, here described comprises,
upon submission of a tobacco cigarette user identifier ID at a user smoker terminal 12, performing a self-reporting, i.e. procedure 300 of tobacco cigarette user activity information, i.e. self-report Q, comprising information, such as NCC, NDC, regarding the smoking activity in a given period, in particular daily. Performing the procedure 300 may include completing the self-report Q, partially completing, or leaving it blank, i.e. not answering any of the questions at operations 320-350.

Upon execution of said self-reporting 300, which as said may include not answering the questions at operations 320-350, the method in general includes sending 420 corresponding tobacco cigarette user activity information, i.e. the self-report Q comprising information such as NCC, NDC, regarding the smoking activity in a given period, e.g. daily, from the user smoker terminal 12, through the front-end application there stored, to a remote computer server 14 through a communication link 13.

The server 14 through the back-end application BE can perform operations of:
storing 510 said tobacco cigarette user activity information, i.e. self-report Q comprising information (NCC, NDC) regarding the smoking activity in a given period from the user smoker terminal 12 in a data base 14b at the remote server (14), in particular in records ordered by identifier ID and period, e.g. day,
performing 520 one or more control procedures, such as procedures 600, 700, 800 on the basis of the tobacco cigarette user activity information Q comprising information NCC, NDC regarding the smoking activity in a given period from the user smoker terminal 12 stored in said data base (14), at least one of said one or more control procedures 600 including evaluating a number NCC of cigarettes smoked in the given period, in particular daily,
evaluating said number of cigarettes NCC and in case said number of cigarettes NCC does not match a given criterion, generating 520 at the server 14 a corresponding alert, e.g. alert 620,
sending 530 said alert, e.g. 620 to a controlling entity terminal 15, e.g. the mobile terminal of a HCP, in particular by a push notification.

Such solution advantageously provides a tracking of the smoking activity using a self-reporting, which is however subject to automatic control procedures, thus either indicating the compliance to protocols or readily, in particular, by push notification, indicating to the controller the non-compliance, so that the controller can take action.

As mentioned, a web interface 14c which can be accessed by the controller terminals 15, in particular through a respective controller link 17, is provided. Such web interface 14c can provide a graphical interface, available at a given URL address, accessible to the controller with a specific controller identification IDP, which allows access to controller functions only, i.e. to HCP.

Therefore, after step 520, a step 540 of publishing the data on the web interface 14c may be also performed. Such step 540 may also include to access to the self-report data Q to process data of the tobacco cigarette users further than alert notifications related and to publish the results on the web interface 14c. For instance, statistics, metrics and historical data calculated on the basis of the self-report data in database 14, regarding either the single user 11 or the population of tobacco cigarette users 11 can be processed and shown on the web interface 14c, along with other information, such as anagraphical data or anamnestic information which may be stored in the database 14b or in another database accessible by the backend application BE.

For instance, on the web interface 14c it may be shown the percentage of tobacco cigarette users 11 being tracked which are compliant, partially compliant or not compliant.

The solution according to the various embodiments here described allows to obtain the following advantages.

The solution here described allows a close tracking of a large number of tobacco cigarette users by one or more controllers, e.g. health care providers, or HCP, in particular such as investigators, doctors, researchers, by implementing a method for self-reporting information about the tobacco cigarettes, or TCs, consumption and/or nicotine delivery devices, or NDDs, usage on a periodic basis by means of a front-end of an application at a user terminal, which communicates the corresponding data to a back-end application, which stores such data and processes such data in order to check for violation of given rules and issue corresponding notifications to terminals of the controllers. This allows the controller to checks the level of adherence to the instructions given to users. As mentioned, combination of self-reporting using the application in the user terminal and control performed at the backend, followed by notifications to the controlling entity, allows the controlling entity, e.g., doctor, to follow the results of the application of the protocol and to readily intervene in case of non-compliance with the protocol.

Also, the front-end application is advantageously equipped with further self-reporting functions regarding for instance glycemia and insurgence of health problems. Under this view, also the solution allows direct contact with the doctor if there is a necessity.

The solution here described also advantageously incorporates a daily step count function.

The solution here described also advantageously allows to review the goals set by the doctor and to monitor their progress.

Of course, without prejudice to the principle of the embodiments, the details of construction and the embodiments may vary widely with respect to what has been described and illustrated herein purely by way of example, without thereby departing from the scope of the present embodiments, as defined the ensuing claims.

Although the exemplary embodiment is directed in particular to diabetic tobacco cigarette users, hence the check on the glycemia, in variant embodiments the method described can be directed only to check the smoke reduction, or include further checks in the self-report directed to tobacco cigarette users affected by schizophrenia, chronic obstructive pulmonary disease, cardiovascular disease, or other categories of tobacco cigarette users (including people concerned about their oral health).

## Claims

1. A method for tracking activity of tobacco cigarette users (11) making use of tobacco cigarettes and/or nicotine delivery systems, said use being tracked by a controlling entity (15), comprising,
upon submission of a tobacco cigarette user identifier (ID) at a user smoker terminal (12), performing a self-reporting (300) of tobacco cigarette user activity information (Q) comprising information (NCC, NDC) regarding the smoking activity in a given period, in particular daily, in particular in the day before the self-reporting,
upon execution of said self-reporting (300) sending (420) corresponding tobacco cigarette user activity information (Q) comprising information (NCC, NDC) regarding the smoking activity in a given period from the user smoker terminal (12) to a remote computer server (14) through a communication link (13),
storing said tobacco cigarette user activity information (Q) comprising information (NCC, NDC) regarding the smoking activity in a given period from the user smoker terminal (12) in a data base (14b) at the remote server (14),
performing (520) one or more control procedures (600, 700, 800) on the basis of the tobacco cigarette user activity information (Q) comprising information (NCC, NDC) regarding the smoking activity in a given period from the user smoker terminal (12) stored in said data base (14), at least one of said one or more control procedures (600) including evaluating a number (NCC) of cigarettes smoked in the given period, in particular daily,
evaluating said number of cigarettes (NCC) and in case said number of cigarettes (NCC) does not match a given criterion, generating (520) at the server (14) a corresponding alert (620),
sending (530) said alert (620) to a controlling entity terminal (15).

2. Method according to claim 1, **characterized in that** said server includes a web interface (14c) accessible from said controlling entity terminal, said web interface (14c) being configured to display data stored of said database (14) or processing of said data and/or display said alert (620), upon access.

3. Method according to claim 1, **characterized in that** said sending (530) said alert (620) to a controlling entity terminal (15) is performed by push notification (N) .

4. Method according to claim 1, **characterized in that** said information regarding the smoking activity in a given period includes information (NDC) regarding usage of an electronic nicotine delivery device.

5. Method according to claim 1, **characterized in that** said evaluating if said number of cigarettes (NCC) matches a given criterion includes evaluating a variation of said number of cigarettes (NCC) with respect to a reference baseline number (NC) of smoked cigarettes, in particular stored during an initialization phase before the first self-reporting operation,

6. Method according to claim 5 any of the preceding claims, **characterized in that** said evaluating if said number of cigarettes (NCC) matches a given criterion includes evaluating if a reduction of the smoked number of cigarettes (NCC) in the given period with respect to the baseline number (NC) greater of a given threshold value occurs.

7. Method according to claim 5 or 6, **characterized in that** includes issuing an alert message if said reduction occurs in an observation period comprising a plurality of given periods, in particular a number of consecutive given periods and/or a number of periods between or from a determined event, in particular a medical visit.

8. Method according to any of the previous claims, **characterized in that** said self-reporting includes reporting (310) whether the tobacco cigarette user (11) has performed a glycemia check in the given period.

9. Method according to any of the previous claims, **characterized in that** said self-reporting includes self-reporting (340) whether the tobacco cigarette user (11) has reported a health status in the given period.

10. A system for tracking activity of tobacco cigarette users (11) making use of tobacco cigarettes and/or nicotine delivery systems, said use being tracked by a controlling entity (15) comprising
at least a user terminal (12) comprising a front end of a software application (FE) configured for inputting user data (Q),
a remote server (14) comprising a back-end of said software application (BE) coupled through a communication link (13) to said front end application (BE) in the user terminal (12) and a data base (14b) accessible for writing or reading data by the back-end application (BE),
said system being configured to perform the method of any of claims 1 to 9.

11. The system of claim 10, wherein said back-end application (BE) comprises a user interface (14c) to access, from the node (14) and/or a terminal (15) external to said node (14), inputted user data and/or processed user data and to issue signals, obtained from said inputted user data and/or processed user, which are also accessible on said user interface,

12. A computer program product that can be loaded into the memory of at least one computer and comprises parts of software code that are able to execute the steps of the method of any of claims 1 to 9 when the product is run on at least one computer.
